# EUROPEAN PATENT SPECIFICATION

(11) **EP 0 320 842 B1**
(45) Date of publication and mention of the grant of the patent: **28.07.1993**
(21) Application number: 88120709.6
(22) Date of filing: 12.12.1988
(51) Int. Cl.: G01N 33/543, G01N 33/551, C12Q 1/68

(54) **Method for binding biological reagents to porous supports**
Verfahren zur Bindung von biologischen Reagenzien an poröse Träger
Méthode pour attacher les réactifs biologiques à des supports poreux

(30) Priority: 18.12.1987 US 134644
(43) Date of publication of application: 21.06.1989
(73) Proprietor: W.R. Grace & Co.-Conn., New York, New York 10036 (US)
(72) Inventor: Rudolph, Julie Liao, Carlisle Massachusetts 01741 (US)
(74) Representative: UEXKÜLL & STOLBERG

(56) References cited:
- EP-A- 0 212 670
- WO-A-84/03150

## Description

### BACKGROUND OF THE INVENTION

This invention relates generally to the field of immobilization of biological agents onto solid supports for use in diagnostic assays or other analytical testing. More specifically, a method has been found for directly applying a biological agent, such as an antibody or antigen, onto a support, particularly where the support already is part of an assembled device. The present method utilizes a binding aid in conjunction with the biological agent.

Diagnostic and other assay devices frequently utilize a small dot of antibody or other biological agent on a porous structure, such as a microporous membrane. In the assembled device, the porous structure typically is positioned over an absorbent wick material which facilitates flow of the test and reagent fluids through the treated porous structure during the assay. Difficulties have been encountered in applying and immobilizing the biological agent to the desired portion of the porous structure, particularly in the presence of the absorbent material. If the biological agent solution is applied directly, it will be absorbed onto the wick, rather than remaining deposited on the porous structure.

### SUMMARY OF THE INVENTION

The method of this invention allows antibodies or other biological agents to be bound directly to a porous structure, even in the presence of an absorbent material which is in contact with the porous structure. The biological agent is viscosified by mixing with a binding aid and the viscosified biological agent is applied to the porous structure and dried.

It is a primary object of this invention to provide a simplified method for depositing and immobilizing a biological agent on a porous structure suitable for use in diagnostic or other assays. It is intended to provide flexibility in assembling devices to be used in such assays, in that the biological agent can be applied either to the porous structure alone, or to the porous structure as it is in the assembled device, or at any time during assembly of the device.

It is an additional object to provide a method which may be used both with low and high molecular weight biological agents.

Another important object of the invention is to apply and immobilize sufficient biological agent for the assay, while avoiding over- or underapplication of the agent. It is also intended that the method avoid or prevent nonspecific binding which would affect the background color observed during the assay.

A further object is to provide a method which enables the biological agent to be concentrated and immobilized in a small, well-defined circular area on the porous support.

Finally, it is intended that the technique of this invention yield immobilized antibodies, etc., which retain their biological activity.

### DETAILED DESCRIPTION OF THE INVENTION

Biological agents may be directly applied to porous structures by mixing the biological agent with a suitable binding aid to form a homogeneous solution. Even where the porous structure is in contact with an absorbent material, the biological agent solution applied by this method will remain localized on and become immobilized on the porous structure without passing through to the absorbent material.

A variety of biological agents can be applied in this manner. Of primary interest for the preparation of diagnostic assay devices will be antibodies, either polyclonal or monoclonal. Antigens also may be immobilized by this method, in particular, low molecular weight antigens which cannot be directly immobilized onto porous supports by conventional means. It frequently is difficult to immobilize low molecular weight agents, both because they tend to flow through the porous support and because they tend to have a low affinity for the support surface. Nonetheless, it is very desirable to immobilize them on a porous support. Immobilized antigens may be needed, for example, where a positive control is desired in a particular assay. Other biological agents which may be immobilized by this method are RNA or DNA probes, proteins, and particle suspensions of any of these agents. Low and high molecular weight biological agents may be used in this invention.

As one specific example, a solution of a binding aid and the antibody anti-human chorionic gonadotropin (anti-HCG) may be deposited on a porous structure. If a positive test control is desired, a solution of a binding aid and human chorionic gonadotropin (HCG) itself (the antigen) may be deposited in an adjacent portion of the porous structure. This embodiment would be useful for diagnosing human pregnancy. Many other embodiments are possible, depending on selection of the desired biological agent.

The term "binding aid" is used herein to designate the ingredient(s) added to the biological agent prior to deposition on the porous support. These ingredients are not necessarily binding agents in the conventional sense of the term. Rather, the added ingredients described herein aid in the inherent ability of the biological agent to become immobilized on the porous support, by reducing or eliminating significant amounts of flow-through or diffusion. This is accomplished chiefly by increasing the viscosity of the solution containing the biological agent. Thus, the binding aids of this invention selected from the group consisting of a cellulose derivative, a starch, a polysaccharide, gelatin, agar, a polyelectrolyte or any mixture thereof to form a homogeneous viscous solution do not themselves attach or link the biological agent to the support, but serve to localize the biological agent so that it becomes immobilized in the desired position. This localization also ensures that the entire deposited quantity of biological agent is immobilized on the support.

Suitable binding agents are those which do not inactivate the biological agent when they are contacted as described herein. That is, the binding aid should not bind to or react with the biological agent in such a way as to block, destroy or otherwise inactivate the biologically active site. In addition, the binding aid must be non-binding to the detecting reagent to be used in the assay. For example, where the detecting reagent is an antibody-enzyme conjugate such as commonly used in immunoenzymetric assays, the conjugate must be susceptible to binding only to the antibody. Binding of the reagent to the binding aid would give false results.

It has been found that the binding aids of this invention are non-binding to enzyme conjugates commonly used in this field. This is determined by performing a negative control experiment in which the binding aid is deposited on the support without the biological agent, then exposed to the enzyme conjugate and substrate. If there is no color change, the binding aid is suitable for use with the reagents. If there is a color change, a different binding aid must be selected for that particular assay system.

Useful binding aids include cellulose derivatives (e.g., hydroxy-ethyl- or hydroxypropylcellulose, carboxymethylcellulose, etc.), starches, polysaccharides, gelatin, agar, polyelectrolytes (e.g., polyacrylic acids) and the like. Other materials which are used to increase the viscosity of liquids may be used. It is preferred that the binding aid be water soluble so that a solution with the biological agent may be formed. It is also preferred that the binding aid exhibit low nonspecific protein binding over the time period during which the assay is conducted. Mixtures of binding aids may be used.

A solution is prepared by mixing the biological agent and the binding aid so that a homogeneous solution is formed. The biological agent and the binding aid are used in a ratio of between about 1:4 to about 4:1 to localize the solution on the porous support material. Where the binding aids are available in dry or powder form, it is preferred to first make a thick aqueous solution of the binding agent. For example, a 1% solution typically will be suitable. Water is typically used, but other aqueous liquids, such as phosphate buffered saline, may be used if desired. The binding aid solution may be prepared at room temperature, or at slightly elevated temperatures if faster dissolution is desired.

The biological agent/binding aid solution is readily formed under ambient conditions. However, higher or lower temperatures may be selected, as long as they do not inactivate the biological agent. The two components are mixed in a suitable ratio to obtain a thick, viscous solution. The intended concentration of biological agent deposited on the porous support will be fixed by the assay for which it is to be used. Therefore, the ratio of binding aid solution to biological agent solution will be calculated depending on the concentration of both solutions. Volumetric ratios of between about 4:1 and about 1:4 may be used, preferably between about 2:1 and about 1:2. As a rule of thumb, at least about 20% binding aid (as a 1% solution) will be used.

Application of the biological agent/binding aid solution to the porous support may conveniently be conducted at room temperature. Where very wide pore supports are used, it will be preferred to refrigerate the reagents or the combined solution prior to application in order to further increase viscosity. By contrast, elevated temperatures will decrease viscosity and therefore are not preferred.

The biological agent/binding aid solution may be applied to any porous structures which are suited for use in assay devices. Most commonly used are microporous membranes. Nylon membranes are frequently used. Alternatively, membranes of polypropylene, polyurethane, polysulfones, polyacrylates, various polyesters, polyvinylidene difluoride, Teflon (TM) (E. I. duPont de Nemours & Company) or cellulose may be used. Membranes with pore sizes of about 0.05µm or less to about 10.0µm or greater are typically used. Alternatively, a porous ceramic structure may be used. Ceramics used as assay supports typically have about 30 to 60% porosity. The present invention could be used on less porous ceramic, but that ceramic likely would not be suitable for use in this type of assay.

The solution is deposited onto the porous structure in the desired amount. Preferably, a small amount, typically about two to about ten microliters, of the solution is dotted onto the porous support to produce a small, well-defined circular area containing the biological agent. Alternatively, a line or band of the solution may be applied, or any other desired configuration or insignia. Deposition of precise quantities of the biological agent is made possible by this invention. Mixture of the biological agent and the binding aid yield a homogeneous solution which offers the ability to control the quantity of biological agent deposited for each assay, ensuring a good level of quality control for production of the devices.

The deposited biological agent/binding aid solution is dried to immobilize the biological agent on the porous support structure. Air drying at ambient temperatures is preferred, but drying may be done at lower or higher temperatures, if preferred, provided that temperatures are not employed which would denature or otherwise deactivate the biological agent. Vacuum drying may be used, if desired. The support is dried until the biological agent is thoroughly dry, usually at least 2 to 4 hours.

As discussed above, the present method may be used to apply biological agent to device components (i.e., membrane sheets) or to completed or partially completed devices. Despite the porosity of the support, the biological agent remains localized on or near the support surface by using sufficient amounts of the binding aid to achieve this biological agent-localizing effect. Flow-through of the biological agent solution is avoided, thus ensuring that the entire deposited amount of biological agent is retained on the surface of the membrane or other porous structure, as desired. This is particularly advantageous where an absorbent material underlies or is adjacent to the porous structure, which would otherwise act as a wick to pull the biological agent through the porous structure. It is also quite advantageous where wide pore membranes or other wide pore supports are used. Further, the method described here avoids lateral diffusion or bleeding of the solution, which also would dissipate the biological agent. Addition of the binding aid to the biological agent prior to deposit on the porous support results in a discrete, reproducible dot on the test device.

This invention offers yet another advantage with respect to the requirement for "blocking" the assay supports. Blocking is required with conventional membranes because the membranes themselves exhibit high levels of nonspecific protein binding. Nonspecific binding (of, for example, the enzyme conjugate) across the entire support renders the assay inaccurate and unreadible. The supports are therefore contacted with a protein solution (e.g., casein) to thoroughly coat the support, filling or blocking the nonspecific binding sites available on the support surface.

In preparing conventional assay systems, the biological agent first must be immobilized and dried on the support, and then blocked; conventional preparations of the biological agent will not adhere to the blocked support. With the present invention, this sequence is no longer required. The membrane, or other porous support, can be blocked in a wholly separate procedure from immobilization of the biological agent. This enables the supports to be prepared in large quantities, prior to application of the frequently costly biological agent. When the biological agent/binding aid solution of this invention is deposited, the biological agent will become immobilized, notwithstanding prior blocking of the support against nonspecific protein adsorption.

The examples which follow are given for illustrative purposes and are not meant to limit the invention described herein. The following abbreviations have been used throughout in describing the invention:
- HCG: = human chorionic gonadotropin
- mIU: = milli-International Units
- µl: = microliter(s)
- %: = percent
- TM: = trademark

### EXAMPLE I

Beta-human chorionic gonadotropin (beta-HCG) (500 mIU) was mixed with an equal volume of a 1% solution (in water) of Natrosol 250 HR (TM) (Hercules, Inc.) hydroxyethylcellulose and incubated at room temperature for one minute. A small amount of the solution was applied in single dots (2-5 µl each) onto a nylon membrane and onto a cellulose membrane. The membranes were air-dried under ambient conditions for over four hours. The membranes were then blocked with a 0.1% casein solution to prevent nonspecific protein binding on the untreated portion of the membrane. The membranes again were air-dried.

Immobilized beta-HCG was detected on both membranes as a well-defined, distinct blue dot by immunoenzymetric assay. In the assay, the treated membrane was contacted with anti-beta-HCG-antibody-enzyme conjugate, rinsed and then contacted with the enzyme substrate, triggering the color change. In this example, the enzyme conjugate was a peroxidase conjugate and the substrate was tetramethylbenzidene. The results demonstrated the efficacy of a positive control for a beta-HCG diagnostic assay utilizing the method of this invention for immobilization of the antigen (beta-HCG) on nylon and on cellulose microporous membranes.

### EXAMPLE II

The procedures of Example I were repeated, using a polyurethane microporous membrane and omitting the casein blocking step. Similar results were obtained.

### Example III

The procedures of Example I were repeated, using a 3% solution of gelatin in water instead of the Natrosol 250 HR solution. Similar results were obtained.

### Example IV

The procedures of Example I were repeated, using a 2% solution of starch in water instead of the Natrosol 250 HR solution. Similar results were obtained.

### Example V

Anti-beta-HCG antibody was mixed with an equal volume of 1% Natrosol 250 HR (TM) (Hercules, Inc.) hydroxyethylcellulose and incubated for one minute at room temperature. A small amount (5µl) was deposited on nylon and on polyurethane microporous membranes, below and in contact with which were cellulose absorbent pads. The membranes were dried and then the assay conducted as in Example I. The assay reagents were beta-HCG, a beta-HCG-enzyme conjugate and enzyme substrate. The enzyme conjugate used in this example was an alkaline phosphatase conjugate and the substrate was indoxyl phosphate. A well-defined, distinct blue dot was detected on each membrane, as evidence of a positive test result for a beta-HCG assay.

### EXAMPLE VI

The procedures of Example V were repeated except for the substitution of a ceramic cylinder (40% porosity) for the microporous membrane. Similar results were obtained.

## Claims

1. A method for preparing an assay device for an immobilized biologicals assay, comprising:
(a) selecting a porous support material,
(b) mixing a biological agent and a binding aid selected from a cellulose derivative, a starch, a polysaccharide, gelatin, agar, a polyelectrolyte or any mixture thereof to form a homogeneous viscous solution,
(c) applying said solution to said porous support material in an amount effective to immobilize the biological agent in the desired position, and
(d) thoroughly drying the porous support material of step (c).

2. The method of claim 1 in which said porous support material is a microporous membrane or ceramic.

3. The method of claims 1 or 2 in which said biological agent is a polyclonal antibody, a monoclonal antibody, an antigen, a DNA probe, an RNA probe, or a suspension or solution thereof.

4. The method of claims 1 to 3, wherein said cellulose derivative is hydroxyethylcellulose, hydroxypropylcellulose or carboxymethylcellulose.

5. The method of claims 1 to 4 in which said biological agent and said binding aid are used in a ratio of between about 1:4 to about 4:1 to localize the solution on the porous support material.

## Patentansprüche

1. Verfahren zur Herstellung einer Testvorrichtung für einen immobilisierten biologischen Test, der
(a) das Auswählen eines porösen Trägermaterials,
(b) das Mischen eines biologischen Agens und einer Bindungshilfe ausgewählt aus einem Cellulose - Derivat, einer Stärke, einem Polysaccharid, Gelatine, Agar, einem Polyelektolyten oder einer beliebigen Mischung davon zur Bildung einer homogenen, viskosen Lösung,
(c) das Aufbringen der Lösung auf das poröse Trägermaterial in einer ausreichenden Menge, um das biologische Agens in der gewünschten Position zu immobilisieren und
(d) die gründliche Trocknung des porösen Trägermaterials von Stufe (c)
umfaßt.

2. Verfahren nach Anspruch 1, bei dem das poröse Trägermaterial eine mikroporöse Membran oder Keramik ist.

3. Verfahren nach den Ansprüchen 1 oder 2, bei dem das biologische Agens ein polyklonaler Antikörper, ein monoklonaler Antikörper, ein Antigen, eine DNA-Probe, eine RNA-Probe oder eine Suspension oder Lösung davon ist.

4. Verfahren nach den Ansprüchen 1 bis 3, bei dem das Zellulose Derivat Hydroxyethylcellusose, Hydroxypropylcellulose oder Carboxymethylcellulose ist.

5. Verfahren nach den Anprüchen 1 bis 4, bei dem das biologische Agens und die Bindungshilfe in einem Verhältnis von etwa 1:4 bis etwa 4:1 verwendet werden, um die Lösung auf dem porösen Trägermaterial zu lokalisieren.

## Revendications

1. Méthode pour la préparation d'un dispositif d'analyse pour une analyse biologique immobilisée, comprenant
(a) la sélection d'un matériau de support poreux,
(b) le mélange d'un agent biologique et d'un auxiliaire de liaison choisi parmi un dérivé de cellulose, un amidon, un polysaccharide, de la gélatine, de l'agar, un polyélectrolyte et tout mélange de ceux-ci pour former une solution visqueuse homogène,
(c) l'application de ladite solution audit matériau de support poreux en une quantité efficace pour immobiliser l'agent biologique à la position souhaitée, et
(d) le séchage complet du matériau de support poreux de l'étape (c)

2. Méthode de la revendication 1 où ledit matériau de support poreux est une membrane ou une céramique microporeuse

3. Méthode des revendications 1 ou 2 où ledit agent biologique est un anticorps polyclonal, un anticorps monoclonal, un antigène, une sonde d'ADN, une sonde d'ARN, ou une suspension ou solution de ceux-ci.

4. Méthode des revendications 1 à 3 où ledit dérivé de cellulose est hydroxyéthylcellulose, hydroxypropylcellulose ou carboxyméthylcellulose.

5. Méthode selon les revendications 1 à 4 où ledit agent biologique et ledit auxiliaire de liaison sont utilisés à un rapport compris entre environ 1:4 et environ 4:1 pour localiser la solution sur un matériau de support poreux.
